# EUROPÄISCHE PATENTSCHRIFT

(11) **EP 4 525 781 B1**
(45) Veröffentlichungstag und Bekanntmachung des Hinweises auf die Patenterteilung: **13.08.2025**
(21) Anmeldenummer: 23723606.2
(22) Anmeldetag: 12.05.2023
(51) Int. Cl.: A61F 2/16, A61F 9/00, A61K 9/00

(54) **MEDIKAMENTENSPEICHER FÜR EINE INTRAOKULARLINSE UND INTRAOKULARLINSE MIT EINEM SOLCHEN MEDIKAMENTENSPEICHER**
MEDICAMENT RESERVOIR FOR AN INTRAOCULAR LENS, AND INTRAOCULAR LENS COMPRISING SUCH A MEDICAMENT RESERVOIR
RÉSERVOIR DE MÉDICAMENT POUR LENTILLE INTRAOCULAIRE ET LENTILLE INTRAOCULAIRE COMPRENANT UN TEL RÉSERVOIR DE MÉDICAMENT

(30) Priorität: 20.05.2022 DE 102022112804
(43) Veröffentlichungstag der Anmeldung: 26.03.2025
(73) Patentinhaber: Carl Zeiss Meditec AG, 07745 Jena (DE)
(72) Erfinder: DAMM, Niklas, 73447 Oberkochen (DE); WOLFSTEIN, André, 73447 Oberkochen (DE)
(74) Vertreter: PATERIS Patentanwälte PartmbB
(86) Internationale Anmeldenummer: PCT/EP2023/062818
(87) Internationale Veröffentlichungsnummer: WO 2023/222552

(56) Entgegenhaltungen:
- WO-A1-2008/113043
- WO-A1-2020/264425
- WO-A1-2022/076456

## Beschreibung

Die Erfindung betrifft einen Medikamentenspeicher für eine Intraokularlinse und eine Intraokularlinse mit einem solchen Medikamentenspeicher. WO 2022/076456 A1 offenbart eine Intraokularlinse mit Medikamentenabgabevorrichtungen. WO 2020/264425 A1 offenbart okulare Abgabevorrichtungsverfahren und -systeme. WO 2008/113043 A1 offenbart eine Vorrichtung und ein Verfahren für eine intraokulare Medikamentenabgabe

Bei einer Kataraktbehandlung eines Auges wird eine natürliche Linse durch eine künstliche Intraokularlinse ersetzt. Dazu wird herkömmlich nur ein kleiner Schnitt in die Hornhaut des Auges eingebracht, der so groß ist, dass eine Spitze eines Injektors durch den Schnitt in das Auge eingeführt werden kann. Nachdem der Schnitt in die Hornhaut eingebracht wurde, wird die natürliche Linse des Auges üblicherweise mittels Phakoemulsifikation zerkleinert und anschließend aus dem Kapselsack des Auges abgesaugt. Danach wird die Intraokularlinse mittels des Injektors in das Auge eingesetzt.

Nach der Kataraktbehandlung werden herkömmlich Medikamente, wie beispielsweise Antibiotika und/oder Entzündungshemmer, in das Auge eingebracht. Beispielsweise kann an der Intraokularlinse ein Medikamentenspeicher angebracht sein, der zusammen mit der Intraokularlinse in das Auge eingebracht wird. Wenn die Intraokularlinse via die Spitze in das Auge eingesetzt wird, sollte dabei der Medikamentenspeicher sich nicht von der Intraokularlinse lösen. US 2021 / 0 267 751 A1 offenbart eine intraokulare Medikamentenlieferungsplattform. US 2014 / 0 148 900 A1 offenbart intraokulare Vorrichtungen, an denen ein Medikamentenlieferungskonstrukt angebracht ist.

Aufgabe der Erfindung ist es daher, einen Medikamentenspeicher und eine Intraokularlinse mit dem Medikamentenspeicher zu schaffen, wobei der Medikamentenspeicher, wenn der Medikamentenspeicher an der Intraokularlinse angebracht ist, sich nicht von der Intraokularlinse löst.

Der erfindungsgemäße Medikamentenspeicher für eine Intraokularlinse weist ein Medikament, ein Durchgangsloch, eine erste Medikamentenspeicheraussparung, die mit dem Durchgangsloch kommuniziert, einen ersten Steg, der die erste Medikamentenspeicheraussparung begrenzt, eine erste Stirnfläche, die das Durchgangsloch begrenzt, und eine zweite Stirnfläche auf, die der ersten Stirnfläche abgewandt angeordnet ist und das Durchgangsloch begrenzt, wobei das Durchgangsloch in einem Bereich der ersten Stirnfläche ein erstes Längsende und in einem Bereich der zweiten Stirnfläche ein zweites Längsende aufweist, wobei der Medikamentenspeicher eine Verlagerungsrichtung aufweist, die von dem ersten Längsende zu dem zweiten Längsende gerichtet ist, wobei die erste Medikamentenspeicheraussparung ausgebildet ist, dass das Durchgangsloch via die erste Medikamentenspeicheraussparung in einer auf die Verlagerungsrichtung bezogene Medikamentenspeicherradialrichtung von außerhalb des Medikamentenspeichers zugänglich ist. Der Medikamentenspeicher ist mit einem Haptikarm einer Intraokularlinse koppelbar, indem ein Längsende des Haptikarms, das einem Optikkörper der Intraokularlinse abgewandt angeordnet ist, in dem Durchgangsloch angeordnet wird und anschließend der Medikamentenspeicher in der Verlagerungsrichtung und näher an den Optikkörper heran verlagert wird. Indem danach ein Teil des Haptikarms in die erste Medikamentenspeicheraussparung eingebracht wird, beispielsweise indem der Haptikarm einen Vorsprung aufweist, der eingerichtet ist, in die erste Medikamentenspeicheraussparung einzugreifen, kann der Medikamentenspeicher besonders fest an dem Haptikarm angebracht werden. Dadurch ist eine Wahrscheinlichkeit gering, dass der Medikamentenspeicher sich von der Intraokularlinse löst, insbesondere wenn die Intraokularlinse via eine Spitze eines Injektors in den Kapselsack eines Auges eingesetzt wird.

Die Verlagerungsrichtung ist bevorzugt parallel zu einer Normalen der ersten Stirnfläche und/oder parallel zu einer Normalen der zweiten Stirnfläche angeordnet.

Es ist bevorzugt, dass der erste Steg einen Teil der ersten Stirnfläche bildet. Besonders bevorzugt begrenzt der erste Steg die erste Medikamentenspeicheraussparung in einer Richtung, die entgegen der Verlagerungsrichtung orientiert ist.

Es ist bevorzugt, dass die zweite Stirnfläche in einem Bereich der ersten Medikamentenspeicheraussparung unausgebildet ist, so dass die erste Medikamentenspeicheraussparung in der Verlagerungsrichtung von außerhalb des Medikamentenspeichers zugänglich ist. Dadurch ist es möglich, wenn der Haptikarm in dem Durchgangsloch angeordnet ist, den Haptikarm durch eine Schwenkbewegung des Medikamentenspeichers in die erste Medikamentenspeicheraussparung einzubringen.

Der Medikamentenspeicher weist bevorzugt eine zweite Medikamentenspeicheraussparung, die mit dem Durchgangsloch kommuniziert, und einen zweiten Steg auf, der die zweite Medikamentenspeicheraussparung begrenzt, wobei die zweite Medikamentenspeicheraussparung ausgebildet ist, dass das Durchgangsloch via die zweite Medikamentenspeicheraussparung in einer weiteren auf die Verlagerungsrichtung bezogene Medikamentenspeicherradialrichtung von außerhalb des Medikamentenspeichers zugänglich ist. Dadurch, dass der Haptikarm in der zweiten Medikamentenspeicheraussparung, insbesondere indem ein weiterer Vorsprung des Haptikarms in die zweite Medikamentenspeicheraussparung eingreift, angeordnet wird, kann der Medikamentenspeicher noch fester an dem Haptikarm angebracht werden.

Die erste Medikamentenspeicheraussparung und die zweite Medikamentenspeicheraussparung sind bevorzugt beabstandet voneinander angeordnet.

Der zweite Steg bildet bevorzugt einen Teil der zweiten Stirnfläche. Besonders bevorzugt begrenzt der zweite Steg die zweite Medikamentenspeicheraussparung in der Verlagerungsrichtung.

Es ist bevorzugt, dass die erste Stirnfläche in einem Bereich der zweiten Medikamentenspeicheraussparung unausgebildet ist, so dass die zweite Medikamentenspeicheraussparung entgegen der Verlagerungsrichtung von außerhalb des Medikamentenspeichers zugänglich ist. Dadurch ist es vorteilhaft möglich, durch die Schwenkbewegung den Haptikarm gleichzeitig in die erste Medikamentenspeicheraussparung und die zweite Medikamentenspeicheraussparung einzubringen.

Es ist bevorzugt, dass der Medikamentenspeicher eingerichtet ist, das Medikament kontinuierlich abzugeben. Insbesondere kann der Medikamentenspeicher eingerichtet sein, dabei biologisch abgebaut zu werden. Dazu kann der Medikamentenspeicher eine Matrix, in das das Medikament eingebracht sein kann, mit einem Copolymer aufweisen, das von einem ersten Monomer und einem zweiten Monomer gebildet ist. Das erste Monomer kann ein Caprolacton sein und das zweite Monomer kann ausgewählt sein aus der Gruppe Laktid, Glykolid und/oder Trimethylencarbonat. Ein Beispiel für eine Matrix, in die das Medikament eingebracht sein kann und die nicht biologisch abbaubar ist, ist ein polymerisiertes Hydroxyethylmethacrylat.

Das Medikament kann beispielsweise ein Antibiotikum, wie beispielsweise Moxifloxacin, und/oder einen steroidalen Entzündungshemmer wie beispielsweise Dexamethason, und/oder einen nichtsteroidalen Entzündungshemmer, wie beispielsweise ein nichtsteroidales Antirheumatikum wie zum Beispiel Diclofenac, aufweisen. Das Medikament kann beispielsweise zusätzlich oder alternativ eine diagnostische Substanz, wie beispielsweise ein Kontrastmittel, aufweisen.

Der Medikamentenspeicher kann beispielsweise einen einzigen Werkstoff aufweisen, der die Matrix mit dem in der Matrix eingebrachten Medikament aufweist oder aus der Matrix mit dem in der Matrix eingebrachten Medikament besteht.

Der Medikamentenspeicher weist bevorzugt einen ersten Werkstoff, der die Matrix mit dem in der Matrix eingebrachten Medikament aufweist, und einen zweiten Werkstoff auf, der verschieden von dem ersten Werkstoff ist sowie den ersten Steg und insbesondere den zweiten Steg bildet. Der zweite Werkstoff ist bevorzugt nicht biologisch abbaubar. Dazu kann der zweite Werkstoff ausgewählt sein aus der Gruppe Polymethylmethacrylat, polymerisiertes Hydroxyethylmethacrylat, Polypropylen, Silikon, Acrylat-Copolymer.

Der Medikamentenspeicher weist bevorzugt eine erste Deckplatte auf, die von dem zweiten Werkstoff gebildet ist und die erste Stirnfläche bildet. Der Medikamentenspeicher kann zudem eine zweite Deckplatte aufweisen, die von dem zweiten Werkstoff gebildet ist und die zweite Stirnfläche bildet.

Die erfindungsgemäße Intraokularlinse weist einen Optikkörper, einen Haptikarm, der an dem Optikkörper befestigt ist, und den erfindungsgemäßen Medikamentenspeicher oder eine bevorzugte Ausführungsform des erfindungsgemäßen Medikamentenspeichers auf, wobei der Haptikarm in dem Durchgangsloch angeordnet ist und in der ersten Medikamentenspeicheraussparung angeordnet ist.

Es ist bevorzugt, dass der Haptikarm gekrümmt ausgebildet ist. Besonders bevorzugt ist der Haptikarm C-förmig oder J-förmig ausgebildet.

Der Medikamentenspeicher ist bevorzugt mittels eines Formschlusses und/oder einer Presspassung an dem Haptikarm befestigt. Dadurch ist der Medikamentenspeicher besonders fest an dem Haptikarm angeordnet.

Im Folgenden wird anhand der beigefügten schematischen Zeichnungen die Erfindung näher erläutert. Es zeigen
Figur 1 eine Draufsicht auf eine in einem Kapselsack angeordnet gezeichnete erfindungsgemäße Intraokularlinse,
Figur 2 eine perspektivische Ansicht eines nicht zur Erfindung gehörenden Medikamentenspeichers,
Figur 3 eine perspektivische Ansicht eines ersten erfindungsgemäßen Medikamentenspeichers,
Figur 4 eine perspektivische Ansicht eines zweiten erfindungsgemäßen Medikamentenspeichers,
Figur 5 eine Seitenansicht auf eine erfindungsgemäße Intraokularlinse,
Figur 6 einen Haptikarm und einen erfindungsgemäßen Medikamentenspeicher, der beabstandet von dem Haptikarm angeordnet ist,
Figur 7 den Haptikarm und den Medikamentenspeicher aus Figur **6****,** wobei der Medikamentenspeicher auf den Haptikarm verlagert ist, und
Figur 8 den Haptikarm und den Medikamentenspeicher aus Figuren 6 und **7****,** die in einem Kapselsack angeordnet gezeichnet sind, wobei ein erster Steg des Haptikarms in einer ersten Axialaussparung des Haptikarms und ein zweiter Steg des Haptikarms in einer zweiten Axialaussparung des Haptikarms angeordnet sind.

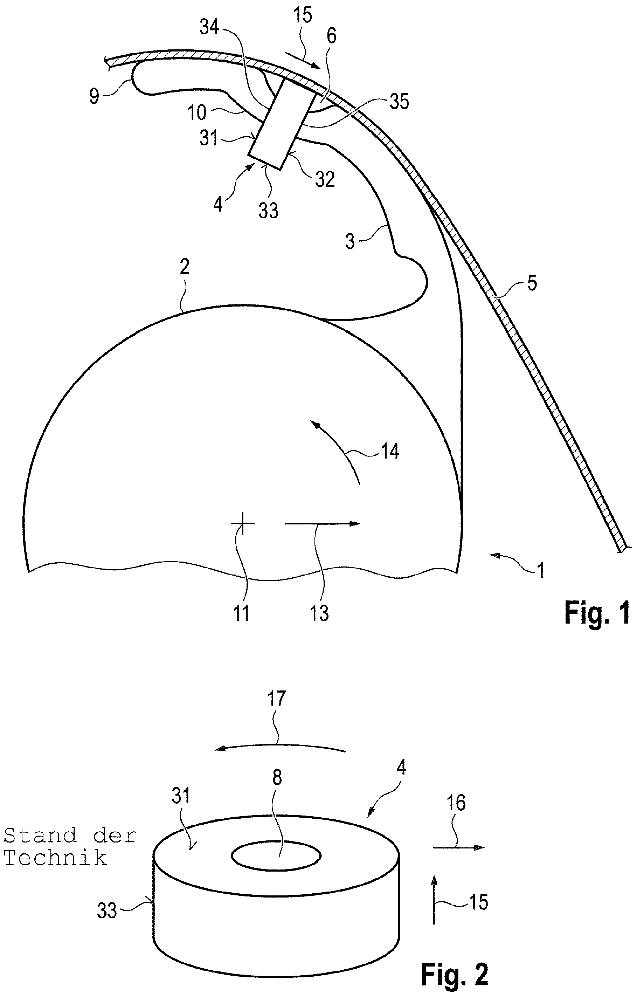

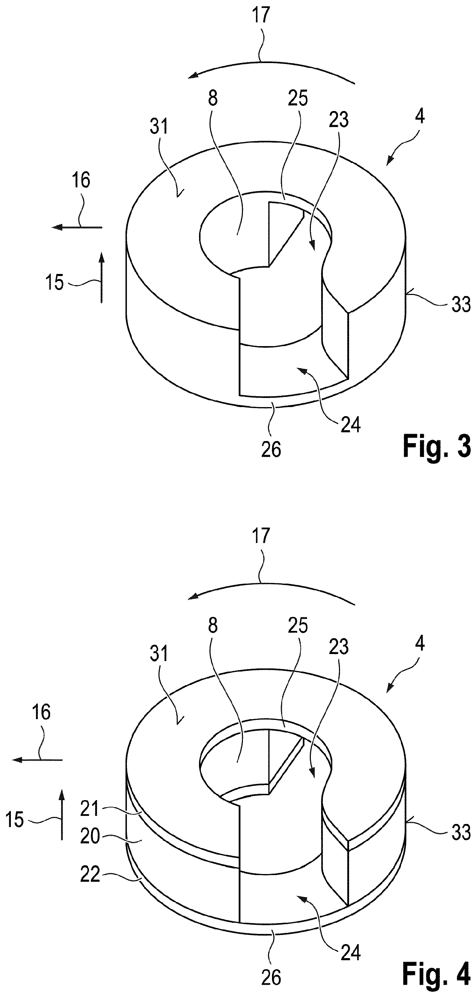

Wie es aus Figuren 3 und 4 ersichtlich ist, weist ein erfindungsgemäßer Medikamentenspeicher 4 für eine Intraokularlinse 1 ein Medikament, ein Durchgangsloch 8, eine erste Medikamentenspeicheraussparung 23, die mit dem Durchgangsloch 8 kommuniziert, einen ersten Steg 25, der die erste Medikamentenspeicheraussparung 23 begrenzt, eine erste Stirnfläche 31, die das Durchgangsloch 8 begrenzt, und eine zweite Stirnfläche 32 auf, die der ersten Stirnfläche 31 abgewandt angeordnet ist und das Durchgangsloch 8 begrenzt. Das Durchgangsloch 8 weist in einem Bereich der ersten Stirnfläche 31 ein erstes Längsende 34 und in einem Bereich der zweiten Stirnfläche 32 ein zweites Längsende 35 auf. Der Medikamentenspeicher 4 weist eine Verlagerungsrichtung 15 auf, die von dem ersten Längsende 34 zu dem zweiten Längsende 35 gerichtet ist. Die erste Medikamentenspeicheraussparung 23 ist ausgebildet, dass das Durchgangsloch 8 via die erste Medikamentenspeicheraussparung 23 in einer auf die Verlagerungsrichtung 15 bezogene Medikamentenspeicherradialrichtung 16 von außerhalb des Medikamentenspeichers 4 zugänglich ist. Der erfindungsgemäße Medikamentenspeicher 4 unterscheidet sich von dem Medikamentenspeicher 4 aus Figur 2 dahingehend, dass bei dem Medikamentenspeicher 4 aus Figur 2 die erste Medikamentenspeicheraussparung 23 nicht vorgesehen ist.

Der Medikamentenspeicher 4 kann an seiner Außenseite eine Umfangsfläche 33 aufweisen, die zwischen der ersten Stirnfläche 31 und der zweiten Stirnfläche 32 angeordnet ist und insbesondere unmittelbar an die erste Stirnfläche 31 und die zweite Stirnfläche 32 angrenzen kann.

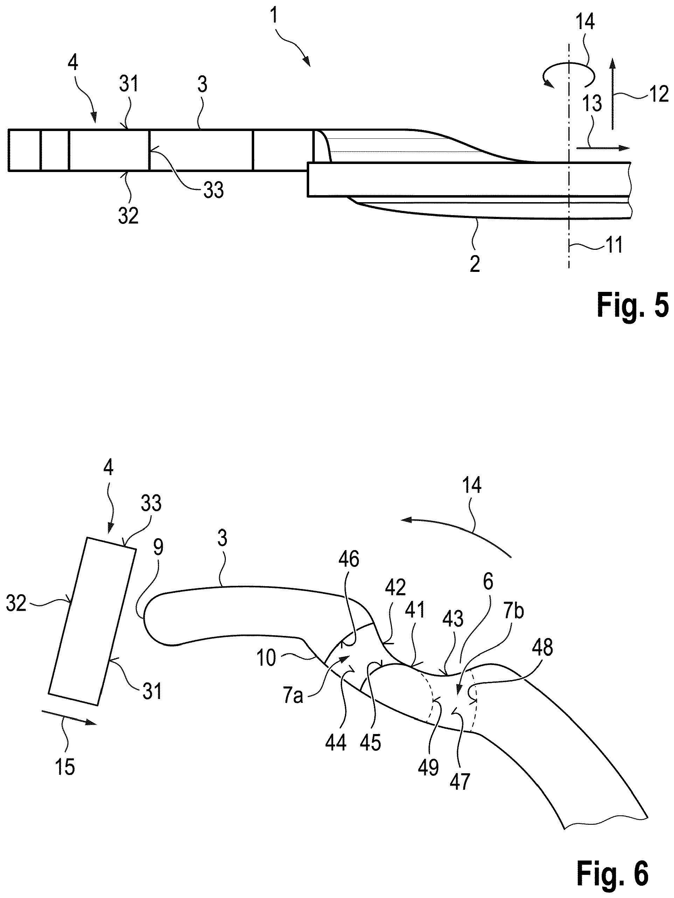

Wie es aus Figuren 1, 5 und 8 ersichtlich ist, weist eine erfindungsgemäße Intraokularlinse 1 einen Optikkörper 2 (vergleiche Figur 1 und 5), einen Haptikarm 3, der an dem Optikkörper 2 befestigt ist, und den Medikamentenspeicher 4 auf, wobei der Haptikarm 3 in dem Durchgangsloch 8 angeordnet ist und in der ersten Medikamentenspeicheraussparung 23 angeordnet ist.

Der Haptikarm 3 kann gekrümmt ausgebildet sein und insbesondere C-förmig (siehe Figur 1) oder J-förmig ausgebildet sein.

Der Medikamentenspeicher 4 kann beispielsweise mittels eines Formschlusses und/oder einer Presspassung an dem Haptikarm 3 befestigt sein.

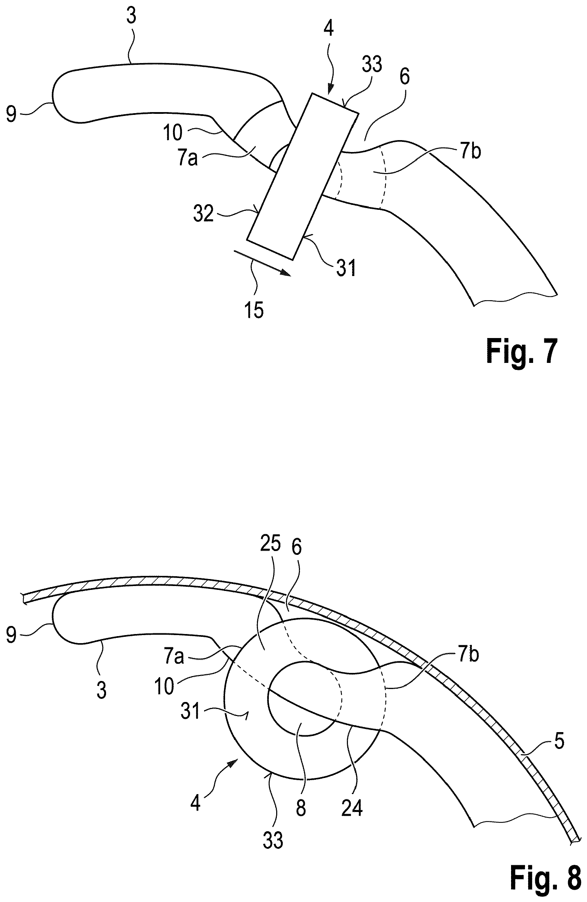

Figuren 1 und 5 zeigen, dass der Optikkörper 2 eine optische Achse 11 aufweisen kann. Der Haptikarm 3 kann eine Radialaussparung 6 aufweisen (vergleiche Figuren 1 und 6 bis 8), in der der Medikamentenspeicher 4 angeordnet ist und die in einer Seite des Haptikarms 3 eingebracht ist, die in einer auf die optische Achse 11 bezogene Radialrichtung 13 außen angeordnet ist. Indem der Medikamentenspeicher 4 in der Radialaussparung 6 angeordnet ist, ist der Medikamentenspeicher 4 besonders fest an dem Haptikarm 3 angeordnet. Dadurch ist eine Wahrscheinlichkeit gering, dass sich der Medikamentenspeicher 4 von dem Haptikarm 3 löst, insbesondere wenn die Intraokularlinse 1 via eine Spitze eines Injektors in einen Kapselsack 5 (vergleiche Figuren 1 und 8) eines Auges injiziert wird. Dadurch, dass die Radialaussparung 6 in der in der Radialrichtung 13 außen liegenden Seite des Haptikarms 3 eingebracht ist, steht der Medikamentenspeicher 4 nicht oder nur geringfügig von dem Haptikarm 3 in der Radialausrichtung 13 nach außen vor. Weil die in der Radialrichtung 13 außen liegende Seite des Haptikarms 3 den Kapselsack 5 kontaktiert, kann somit eine Dezentrierung eines Optikkörpers 2 der Intraokularlinse 1 in dem Auge durch den Medikamentenspeicher 4 verringert oder sogar vermieden werden.

Figuren 1 und 8 zeigen, dass der Medikamentenspeicher 4 in der Radialaussparung 6 versenkt sein kann. Dadurch wird erreicht, dass der Medikamentenspeicher 4, wenn die Intraokularlinse 1 in den Kapselsack 5 eines Auges eingebracht ist, den Kapselsack 5 nicht nach außen, d.h. weg von dem Optikkörper 2, ausbeult, vergleiche Figuren 1 und 8.

Figur 6 zeigt, dass die Radialaussparung 6 von einer ersten Flanke 41 begrenzt sein kann, die ein Verlagern des Medikamentenspeichers 4 entgegen die Radialrichtung 13, d.h. zu der optischen Achse 11 hin, begrenzt. Zudem kann die Radialaussparung 6 von einer zweiten Flanke 42 begrenzt sein, die ein Verlagern des Medikamentenspeichers 4 in einer Umfangsrichtung 14 bezüglich der optischen Achse 11 begrenzt. Außerdem kann die Radialaussparung 6 von einer dritten Flanke 43 begrenzt sein, die ein Verlagern des Medikamentenspeichers 4 in einer Richtung, die entgegen der Umfangsrichtung 14 orientiert ist, begrenzt.

Figuren 3 und 4 zeigen, dass der erste Steg 25 einen Teil der ersten Stirnfläche 31 bilden kann. Figuren 6 bis 8 zeigen, dass der Haptikarm 3 eine erste Axialaussparung 7a aufweisen kann, in der der erste Steg 25 angeordnet ist und die in einer ersten Seite des Haptikarms 3 eingebracht ist, die in einer auf die optische Achse 11 bezogene Axialrichtung 12 außen angeordnet ist. Die zweite Stirnfläche 32 kann in einem Bereich der ersten Medikamentenspeicheraussparung 23 unausgebildet sein, vergleiche Figuren 3 und 4, so dass die erste Medikamentenspeicheraussparung 23 in der Verlagerungsrichtung 15 von außerhalb des Medikamentenspeichers 4 zugänglich ist. Der erste Steg 25 kann die erste Medikamentenspeicheraussparung 23 in einer Richtung begrenzen, die entgegen der Verlagerungsrichtung 15 orientiert ist.

Figur 6 zeigt, dass die erste Axialaussparung 7a von einer ersten Flanke 44 begrenzt sein kann, die ein Verlagern des Medikamentenspeichers 4 entgegen der Axialrichtung 12 begrenzt. Zudem kann die erste Axialaussparung 7a von einer zweiten Flanke 45 begrenzt sein, die ein Verlagern des Medikamentenspeichers 4 entgegen der Umfangsrichtung 14 begrenzt. Außerdem kann die erste Axialaussparung 7a von einer dritten Flanke 46 begrenzt sein, die ein Verlagern des Medikamentenspeichers 4 in der Umfangsrichtung 14 begrenzt.

Die erste Flanke 41 der Radialaussparung 6 und die erste Flanke 44 der ersten Axialaussparung 7a können beispielsweise einen Winkel von 60° bis 120°, insbesondere von 80° bis 100° oder im Wesentlichen 90°, einschließen.

Figuren 3 und 4 zeigen zudem, dass der Medikamentenspeicher 4 eine zweite Medikamentenspeicheraussparung 24, die mit dem Durchgangsloch 8 kommuniziert, und einen zweiten Steg 26 aufweisen kann, der die zweite Medikamentenspeicheraussparung 24 begrenzt, wobei die zweite Medikamentenspeicheraussparung 24 ausgebildet ist, dass das Durchgangsloch 8 via die zweite Medikamentenspeicheraussparung 24 in einer weiteren auf die Verlagerungsrichtung 15 bezogene Medikamentenspeicherradialrichtung 16 von außerhalb des Medikamentenspeichers 4 zugänglich ist. Die erste Medikamentenspeicheraussparung 23 und die zweite Medikamentenspeicheraussparung 24 können beabstandet voneinander angeordnet sein. Insbesondere können die erste Medikamentenspeicheraussparung 23 und die zweite Medikamentenspeicheraussparung 24 so angeordnet sein, dass sie der Krümmung des Haptikarms 3 folgen und somit keine Verformung des Haptikarms 3 bewirken, wenn der Medikamentenspeicher 4 an dem Haptikarm 3 angebracht ist.

Der zweite Steg 26 kann einen Teil der zweiten Stirnfläche 32 bilden. Aus Figuren 6 bis 8 ist zudem ersichtlich, dass der Haptikarm 3 eine zweite Axialaussparung 7b aufweisen kann, in der der zweite Steg 26 angeordnet ist und die in einer auf die optische Achse 11 bezogene Umfangsrichtung 14 versetzt zu der ersten Axialaussparung 7a angeordnet ist sowie die in einer zweiten Seite des Haptikarms 3 eingebracht ist, die entgegen der Axialrichtung 12 außen angeordnet ist und der ersten Seite abgewandt angeordnet ist. Die erste Stirnfläche 31 kann in einem Bereich der zweiten Medikamentenspeicheraussparung 24 unausgebildet ist, so dass die zweite Medikamentenspeicheraussparung 24 entgegen der Verlagerungsrichtung 15 von außerhalb des Medikamentenspeichers 4 zugänglich ist. Der zweite Steg 26 kann die zweite Medikamentenspeicheraussparung 24 in der Verlagerungsrichtung 15 begrenzen.

Figur 6 zeigt, dass die zweite Axialaussparung 7b von einer ersten Flanke 47 begrenzt sein kann, die ein Verlagern des Medikamentenspeichers 4 in der Axialrichtung 12 begrenzt. Zudem kann die zweite Axialaussparung 7b von einer zweiten Flanke 48 begrenzt sein, die ein Verlagern des Medikamentenspeichers 4 entgegen der Umfangsrichtung 14 begrenzt. Außerdem kann die zweite Axialaussparung 7b von einer dritten Flanke 49 begrenzt sein, die ein Verlagern des Medikamentenspeichers 4 in der Umfangsrichtung 14 begrenzt.

Die erste Flanke 41 der Radialaussparung 6 und die erste Flanke 47 der zweiten Axialaussparung 7b können beispielsweise einen Winkel von 60° bis 120°, insbesondere von 80° bis 100° oder im Wesentlichen 90°, einschließen.

Die erste Axialaussparung 7a kann unmittelbar an die Radialaussparung 6 angrenzen und/oder die zweite Axialaussparung 7b kann unmittelbar an die Radialaussparung 6 angrenzen, vergleiche Figuren 6 bis 8. Die zweite Axialaussparung 7b kann in der Umfangsrichtung 14 beabstandet von der ersten Axialaussparung 7a angeordnet sein. Figuren 1 und 6 bis 8 zeigen, dass die Intraokularlinse 1 einen Radialvorsprung 10 aufweisen kann, der in der Radialrichtung 13 nach innen von dem verbliebenen Haptikarm 3 absteht und der in einer auf die optische Achse 11 bezogene Umfangsrichtung 14 in dem gleichen Bereich wie die Radialaussparung 6 angeordnet ist.

Der erste Steg 25 kann in der ersten Axialaussparung 7a versenkt sein, so dass der Medikamentenspeicher 4 in der Radialrichtung 13 gesehen in einem Bereich der ersten Seite fluchtend mit dem Haptikarm 3 angeordnet sein kann, vergleiche Figur 5. Der zweite Steg 26 kann in der zweiten Axialaussparung 7b versenkt sein, so dass der Medikamentenspeicher 4 in der Radialrichtung 13 gesehen in einem Bereich der zweiten Seite fluchtend mit dem Haptikarm 3 angeordnet sein kann, vergleiche Figur 5. Dadurch kann ein Lösen des Medikamentenspeichers 4 von dem Haptikarm 3 bei dem Injizieren der Intraokularlinse 1 besonders sicher vermieden werden. Auch ist es problemlos möglich, bei einem Falten der Intraokularlinse 1 vor dem Injizieren der Intraokularlinse 1 den Haptikarm 3 auf den Optikkörper 2 zu befördern und den Optikkörper 2 um den Haptikarm 3 zu falten.

Die Verlagerungsrichtung 15 kann in die Richtung orientiert sein, in der der Medikamentenspeicher 4 zu verlagern ist, um den Medikamentenspeicher 4 in die Radialaussparung 6 zu verlagern. Es ist denkbar, dass die Verlagerungsrichtung 15 parallel zu einer Normalen der ersten Stirnfläche 31 und/oder parallel zu einer Normalen der zweiten Stirnfläche 32 angeordnet ist. Die erste Stirnfläche 31 kann das in der Verlagerungsrichtung 15 gelegene Längsende des Medikamentenspeichers 4 bilden und die zweite Stirnfläche 21 kann das in einer Richtung, die der Verlagerungsrichtung 15 entgegen gerichtet ist, gelegene Längsende des Medikamentenspeichers 4 bilden.

Figuren 6 bis 8 zeigen, wie der Medikamentenspeicher 4 an dem Haptikarm 3 angebracht werden kann. Zuerst ist der Medikamentenspeicher 4 beabstandet von dem Haptikarm 3 angeordnet. Anschließend kann zuerst ein Längsende 9 des Haptikarms 3, das dem Optikkörper 2 abgewandt angeordnet ist, in dem Durchgangsloch 8 angeordnet werden. Durch ein Verlagern des Medikamentenspeichers 4 in der Verlagerungsrichtung 15 kann der Medikamentenspeicher 4 so lange verlagert werden, bis der Medikamentenspeicher 4 in die Radialaussparung 6 gelangt, vergleiche Figur 7. Bei der ersten Ausführungsform des Medikamentenspeichers 4 ist hier die Verlagerung abgeschlossen und es liegt beispielsweise die in Figur 1 gezeigte Anordnung vor. Bei der zweiten und dritten Ausführungsform des Medikamentenspeichers 4 ist noch eine Schwenkung des Medikamentenspeichers 4 erforderlich, so dass der erste Steg 25 in die erste Axialaussparung 7a gelangt und der zweite Steg 26 in die zweite Axialaussparung 7b gelangt, vergleiche Figur 8. Dabei ist denkbar, dass die Verlagerungsrichtung 15 parallel zu der optischen Achse 11 angeordnet ist, siehe Figur 8.

Der Medikamentenspeicher 4 kann nur einen einzigen Werkstoff aufweisen, der das Medikament aufweist, wie es bei der ersten erfindungsgemäßen Ausführungsform des Medikamentenspeichers 4 gemäß Figur 3 der Fall ist.

Alternativ dazu ist denkbar, dass der Medikamentenspeicher 4 einen ersten Werkstoff aufweist, der das Medikament aufweist, und einen zweiten Werkstoff aufweist, der verschieden von dem ersten Werkstoff ist und den ersten Steg 25 bildet, wie es bei der zweiten erfindungsgemäßen Ausführungsform des Medikamentenspeichers 4 gemäß Figur 4 der Fall ist. Dazu kann der Medikamentenspeicher 4 eine erste Deckplatte 21 aufweisen, die von dem zweiten Werkstoff gebildet ist und die erste Stirnfläche 31 bildet. Es ist auch denkbar, dass der zweite Werkstoff den zweiten Steg 26 bildet. Dazu kann der Medikamentenspeicher 4 eine zweite Deckplatte 22 aufweisen, die von dem zweiten Werkstoff gebildet ist und die zweite Stirnfläche 32 bildet. Zwischen der ersten Deckplatte 21 und der zweiten Deckplatte 22 kann ein Mittelteil 20 angeordnet sein, das von dem ersten Werkstoff gebildet ist und insbesondere die Umfangsfläche 33 bildet.

Die erste Deckplatte 21 und/oder die zweite Deckplatte 22 können porös sein. Dadurch ist es möglich, dass das Medikament schneller freigesetzt wird.

Der Medikamentenspeicher 4 kann eine Säule aufweisen, die sich durch den ersten Werkstoff erstrecken und die die erste Deckplatte 21 und die zweite Deckplatte 22 miteinander verbindet. Die Säule kann beispielsweise den zweiten Werkstoff aufweisen und/oder aus dem zweiten Werkstoff bestehen. Es ist auch denkbar, dass mehrere der Säulen vorgesehen sind, die insbesondere alle die erste Deckplatte 21 und die zweite Deckplatte 22 miteinander verbinden.

### Bezugszeichenliste

1 Intraokularlinse
2 Optikkörper
3 Haptikarm
4 Medikamentenspeicher
5 Kapselsack
6 Radialaussparung
7a erste Axialaussparung
7b zweite Axialaussparung
8 Durchgangsloch
9 Längsende
10 Radialvorsprung
11 optische Achse
12 Axialrichtung
13 Radialrichtung
14 Umfangsrichtung
15 Verlagerungsrichtung
16 Medikamentenspeicherradialrichtung
17 Medikamentenspeicherumfangsrichtung
20 Mittelteil
21 erste Deckplatte
22 zweite Deckplatte
23 erste Medikamentenspeicheraussparung
24 zweite Medikamentenspeicheraussparung
25 erster Steg
26 zweiter Steg
31 erste Stirnfläche
32 zweite Stirnfläche
33 Umfangsfläche
34 erstes Längsende
35 zweites Längsende
41 erste Flanke der Radialaussparung
42 zweite Flanke der Radialaussparung
43 dritte Flanke der Radialaussparung
44 erste Flanke der ersten Axialaussparung
45 zweite Flanke der ersten Axialaussparung
46 dritte Flanke der ersten Axialaussparung
47 erste Flanke der zweiten Axialaussparung
48 zweite Flanke der zweiten Axialaussparung
49 dritte Flanke der dritten Axialaussparung

## Patentansprüche

1. Medikamentenspeicher für eine Intraokularlinse (1), mit einem Medikament und einem Durchgangsloch (8), **dadurch gekennzeichnet, dass** der Medikamentenspeicher (4) eine erste Medikamentenspeicheraussparung (23), die mit dem Durchgangsloch (8) kommuniziert, einen ersten Steg (25), der die erste Medikamentenspeicheraussparung (23) begrenzt, eine erste Stirnfläche (31), die das Durchgangsloch (8) begrenzt, und eine zweiten Stirnfläche (32) aufweist, die der ersten Stirnfläche (31) abgewandt angeordnet ist und das Durchgangsloch (8) begrenzt, wobei das Durchgangsloch (8) in einem Bereich der ersten Stirnfläche (31) ein erstes Längsende (34) und in einem Bereich der zweiten Stirnfläche (32) ein zweites Längsende (35) aufweist, wobei der Medikamentenspeicher (4) eine Verlagerungsrichtung (15) aufweist, die von dem ersten Längsende (34) zu dem zweiten Längsende (35) gerichtet ist, wobei die erste Medikamentenspeicheraussparung (23) ausgebildet ist, dass das Durchgangsloch (8) via die erste Medikamentenspeicheraussparung (23) in einer auf die Verlagerungsrichtung (15) bezogene Medikamentenspeicherradialrichtung (16) von außerhalb des Medikamentenspeichers (4) zugänglich ist.

2. Medikamentenspeicher gemäß Anspruch 1, wobei der erste Steg (25) einen Teil der ersten Stirnfläche (31) bildet.

3. Medikamentenspeicher gemäß Anspruch 1 oder 2, wobei die zweite Stirnfläche (32) in einem Bereich der ersten Medikamentenspeicheraussparung (23) unausgebildet ist, so dass die erste Medikamentenspeicheraussparung (23) in der Verlagerungsrichtung (15) von außerhalb des Medikamentenspeichers (4) zugänglich ist.

4. Medikamentenspeicher gemäß einem der Ansprüche 1 bis 3, wobei der Medikamentenspeicher (4) eine zweite Medikamentenspeicheraussparung (24), die mit dem Durchgangsloch (8) kommuniziert, und einen zweiten Steg (26) aufweist, der die zweite Medikamentenspeicheraussparung (24) begrenzt, wobei die zweite Medikamentenspeicheraussparung (24) ausgebildet ist, dass das Durchgangsloch (8) via die zweite Medikamentenspeicheraussparung (24) in einer weiteren auf die Verlagerungsrichtung (15) bezogene Medikamentenspeicherradialrichtung (16) von außerhalb des Medikamentenspeichers (4) zugänglich ist.

5. Medikamentenspeicher gemäß Anspruch 4, wobei der zweite Steg (26) einen Teil der zweiten Stirnfläche (32) bildet.

6. Medikamentenspeicher gemäß Anspruch 4 oder 5, wobei die erste Stirnfläche (31) in einem Bereich der zweiten Medikamentenspeicheraussparung (24) unausgebildet ist, so dass die zweite Medikamentenspeicheraussparung (24) entgegen der Verlagerungsrichtung (15) von außerhalb des Medikamentenspeichers (4) zugänglich ist.

7. Medikamentenspeicher gemäß einem der Ansprüche 1 bis 6, wobei der Medikamentenspeicher (4) nur einen einzigen Werkstoff aufweist, der das Medikament aufweist.

8. Medikamentenspeicher gemäß einem der Ansprüche 1 bis 6, wobei der Medikamentenspeicher (4) einen ersten Werkstoff aufweist, der das Medikament aufweist, und einen zweiten Werkstoff aufweist, der verschieden von dem ersten Werkstoff ist und den ersten Steg (25) bildet.

9. Medikamentenspeicher gemäß Anspruch 8, wobei der Medikamentenspeicher (4) eine erste Deckplatte (21) aufweist, die von dem zweiten Werkstoff gebildet ist und die erste Stirnfläche (31) bildet.

10. Intraokularlinse mit einem Optikkörper (2), einem Haptikarm (3), der an dem Optikkörper (2) befestigt ist, und einem Medikamentenspeicher (4) gemäß einem der Ansprüche 1 bis 9, wobei der Haptikarm (3) in dem Durchgangsloch (8) angeordnet ist und in der ersten Medikamentenspeicheraussparung (23) angeordnet ist.

## Claims

1. Medicament reservoir for an intraocular lens (1), having a medicament and a through-hole (8), **characterized in that** the medicament reservoir (4) has a first medicament reservoir clearance (23) which communicates with the through-hole (8), a first web (25) which delimits the first medicament reservoir clearance (23), a first end face (31) which delimits the through-hole (8), and a second end face (32) which is disposed so as to face away from the first end face (31) and to delimit the through-hole (8), wherein the through-hole (8) in a region of the first end face (31) has a first longitudinal end (34) and in a region of the second end face (32) has a second longitudinal end (35), wherein the medicament reservoir (4) has a displacement direction (15) which is oriented from the first longitudinal end (34) to the second longitudinal end (35), wherein the first medicament reservoir clearance (23) is formed such that the through-hole (8) is accessible from outside the medicament reservoir (4) by way of the first medicament reservoir clearance (23) in a medicament reservoir radial direction (16) in terms of the displacement direction (15).

2. Medicament reservoir according to Claim 1, wherein the first web (25) forms part of the first end face (31).

3. Medicament reservoir according to Claim 1 or 2, wherein the second end face (32) is not formed in a region of the first medicament reservoir clearance (23), so that the first medicament reservoir clearance (23) is accessible from outside the medicament reservoir (4) in the displacement direction (15).

4. Medicament reservoir according to one of Claims 1 to 3, wherein the medicament reservoir (4) has a second medicament reservoir clearance (24) which communicates with the through-hole (8), and a second web (26) which delimits the second medicament reservoir clearance (24), wherein the second medicament reservoir clearance (24) is formed such that the through-hole (8) is accessible from outside the medicament reservoir (4) by way of the second medicament reservoir clearance (24) in a further medicament reservoir radial direction (16) in terms of the displacement direction (15).

5. Medicament reservoir according to Claim 4, wherein the second web (26) forms part of the second end face (32).

6. Medicament reservoir according to Claim 4 or 5, wherein the first end face (31) is not formed in a region of the second medicament reservoir clearance (24), so that the second medicament reservoir clearance (24) is accessible from outside the medicament reservoir (4) counter to the displacement direction (15).

7. Medicament reservoir according to one of Claims 1 to 6, wherein the medicament reservoir (4) comprises only a single material which comprises the medicament.

8. Medicament reservoir according to one of Claims 1 to 6, wherein the medicament reservoir (4) comprises a first material which comprises the medicament, and a second material which is different from the first material and forms the first web (25).

9. Medicament reservoir according to Claim 8, wherein the medicament reservoir (4) has a first cover plate (21) which is formed by the second material and forms the first end face (31).

10. Intraocular lens having an optical body (2), a haptic arm (3) which is fastened to the optical body (2), and a medicament reservoir (4) according to one of Claims 1 to 9, wherein the haptic arm (3) is disposed in the through-hole (8) and disposed in the first medicament reservoir clearance (23).

## Revendications

1. Réservoir de médicament pour une lentille intraoculaire (1), avec un médicament et un trou traversant (8), **caractérisé en ce que** le réservoir de médicament (4) présente un premier évidement de réservoir de médicament (23) qui communique avec le trou traversant (8), une première nervure (25), qui délimite le premier évidement de réservoir de médicament (23), une première surface d'extrémité (31) qui délimite le trou traversant (8), et une deuxième surface d'extrémité (32) qui est agencée à l'opposé de la première surface d'extrémité (31) et qui délimite le trou traversant (8), le trou traversant (8) présentant une première extrémité longitudinale (34) dans une zone de la première surface d'extrémité (31) et une deuxième extrémité longitudinale (35) dans une zone de la deuxième surface d'extrémité (32), le réservoir de médicament (4) présentant une direction de déplacement (15) qui est orientée de la première extrémité longitudinale (34) vers la deuxième extrémité longitudinale (35), le premier évidement de réservoir de médicament (23) étant réalisé de telle sorte que le trou traversant (8) est accessible via le premier évidement de réservoir de médicament (23) dans une direction radiale de réservoir de médicament (16) par rapport à la direction de déplacement (15) depuis l'extérieur du réservoir de médicament (4).

2. Réservoir de médicament selon la revendication 1, la première nervure (25) formant une partie de la première surface d'extrémité (31).

3. Réservoir de médicament selon la revendication 1 ou 2, la deuxième surface d'extrémité (32) n'étant pas réalisée dans une zone du premier évidement de réservoir de médicament (23), de telle sorte que le premier évidement de réservoir de médicament (23) est accessible depuis l'extérieur du réservoir de médicament (4) dans la direction de déplacement (15).

4. Réservoir de médicament selon l'une quelconque des revendications 1 à 3, le réservoir de médicament (4) présentant un deuxième évidement de réservoir de médicament (24) communiquant avec le trou traversant (8) et une deuxième nervure (26) délimitant le deuxième évidement de réservoir de médicament (24), le deuxième évidement de réservoir de médicament (24) étant réalisé de telle sorte que le trou traversant (8) est accessible via le deuxième évidement de réservoir de médicament (24) dans une autre direction radiale de réservoir de médicament (16) par rapport à la direction de déplacement (15) depuis l'extérieur du réservoir de médicament (4).

5. Réservoir de médicament selon la revendication 4, la deuxième nervure (26) formant une partie de la deuxième surface d'extrémité (32).

6. Réservoir de médicament selon la revendication 4 ou 5, la première surface d'extrémité (31) n'étant pas réalisée dans une zone du deuxième évidement de réservoir de médicament (24), de telle sorte que le deuxième évidement de réservoir de médicament (24) est accessible depuis l'extérieur du réservoir de médicament (4) à l'encontre de la direction de déplacement (15).

7. Réservoir de médicament selon l'une quelconque des revendications 1 à 6, le réservoir de médicament (4) ne présentant qu'un seul matériau qui présente le médicament.

8. Réservoir de médicament selon l'une quelconque des revendications 1 à 6, le réservoir de médicament (4) présentant un premier matériau qui présente le médicament et un deuxième matériau qui est différent du premier matériau et forme la première nervure (25).

9. Réservoir de médicament selon la revendication 8, le réservoir de médicament (4) présentant une première plaque de recouvrement (21) qui est formée par le deuxième matériau et forme la première surface d'extrémité (31).

10. Lentille intraoculaire avec un corps optique (2), un bras haptique (3) fixé au corps optique (2) et un réservoir de médicament (4) selon l'une quelconque des revendications 1 à 9, le bras haptique (3) étant agencé dans le trou traversant (8) et étant agencé dans le premier évidement de réservoir de médicament (23).
